(19) Europäisches Patentamt
European Patent Office
Office européen des brevets

(11) EP 4 417 184 A1

(12) EUROPEAN PATENT APPLICATION
published in accordance with Art. 153(4) EPC

(43) Date of publication:
**21.08.2024 Bulletin 2024/34**

(21) Application number: **22880796.2**

(22) Date of filing: **29.09.2022**

(51) International Patent Classification (IPC):
***A61K 8/37*** *(2006.01)* ***A61K 8/86*** *(2006.01)*
***A61Q 17/04*** *(2006.01)*

(52) Cooperative Patent Classification (CPC):
**A61K 8/37; A61K 8/86; A61Q 17/04**

(86) International application number:
**PCT/JP2022/036513**

(87) International publication number:
**WO 2023/063100 (20.04.2023 Gazette 2023/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.10.2021 JP 2021168424**

(71) Applicant: **Shiseido Company, Ltd.**
**Chuo-ku**
**Tokyo 104-0061 (JP)**

(72) Inventors:
- **KANG, Jasmin**
  **Tokyo 104-0061 (JP)**
- **FUJIYAMA, Nozomi**
  **Tokyo 104-0061 (JP)**
- **NAKANE, Toshihiko**
  **Tokyo 104-0061 (JP)**

(74) Representative: **Cabinet Beau de Loménie**
**158, rue de l'Université**
**75340 Paris Cedex 07 (FR)**

# (54) COSMETIC COMPOSITION

(57) Provided is a cosmetic composition in which the effect of improving ultraviolet absorption ability in at least the UVA region is increased by ultraviolet irradiation or sunlight irradiation.

The cosmetic composition includes a polar oil and a compound I having a structure of formula (I) below, wherein the compound I is 0.01 parts by mass or more and 50 parts by mass or less relative to a total of 100 parts by mass of the polar oil and the compound I.

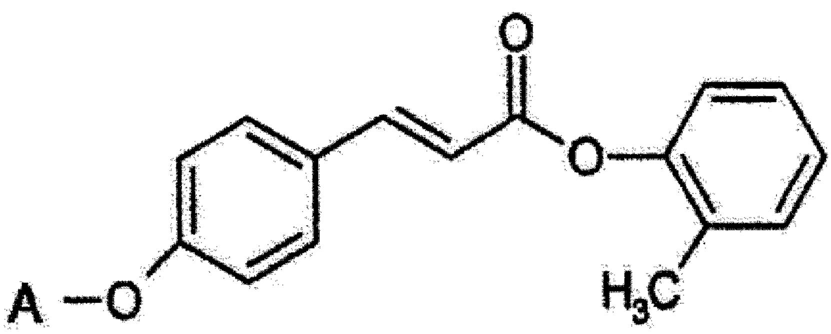

(I)

where -OA represents an alkoxy group.

EP 4 417 184 A1

## Description

FIELD

The present invention relates to a cosmetic composition.

BACKGROUND

Protecting the skin from ultraviolet rays is an important issue in skin care and body care. In addition to medium-wavelength ultraviolet rays (UVB region: wavelength 290 to 320 nm), which are known to cause sunburn and inflammation, long-wavelength ultraviolet rays (UVA region: wavelength 320 to 400 nm) are known to have an effect on the skin (photoaging, etc.).

Conventional cosmetics for ultraviolet protection often contain 2-ethylhexyl p-methoxycinnamate as an ultraviolet absorber. Though 2-ethylhexyl p-methoxycinnamate has the effect of absorbing ultraviolet rays in the UVB region, there is a need for an ultraviolet absorber having better ultraviolet absorption ability in the UVA region.

In connection thereto, the ultraviolet absorber disclosed in Patent Literature 1 has absorption ability in both the UVA region and the UVB region, has an ultraviolet suppression effect in a wide range of wavelengths, and has a previously unseen property: the ultraviolet absorption ability thereof increases in the UVA and UVB regions with the passage of time of ultraviolet irradiation.

More specifically, the ultraviolet absorber of Patent Literature 1 contains, as an active ingredient, a compound represented by formula I:

[Chem 1]

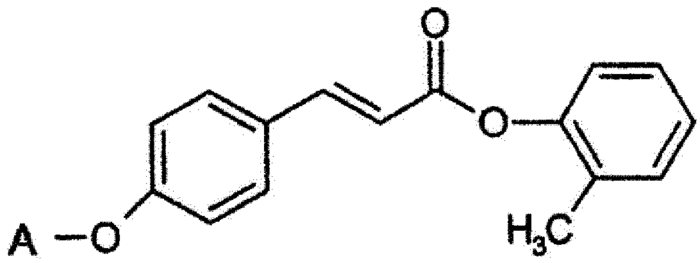

where -OA represents an alkoxy group.

[CITATION LIST]

[PATENT LITERATURE]

[PTL 1] WO 2009/041098

SUMMARY

[TECHNICAL PROBLEM]

In Patent Literature 1, the compound represented by formula I above was blended into an ointment as-is, and the ultraviolet absorption ability thereof was investigated. As a result, it was discovered that the ultraviolet absorption ability in the UVA and UVB regions of an ointment containing the compound represented by formula I increased with increasing ultraviolet irradiation time.

However, conversely, when the compound represented by formula I above was blended into a sunscreen emulsion and the ultraviolet absorption ability thereof was investigated, though the ultraviolet absorption ability thereof did not decrease with increasing irradiation time, no significant increase was observed either.

The present invention aims to improve the situation described above, and an object thereof is to provide a cosmetic composition having an ultraviolet absorption ability significantly improved in at least the UVA region with increasing irradiation time of ultraviolet rays or sunlight.

Note that in the present invention, the degree of increase in the effect of improving ultraviolet absorption ability in at least one of the UVA region and the UVB region by ultraviolet irradiation or sunlight irradiation can be evaluated in

accordance with the ratio of ultraviolet absorbance after irradiation to ultraviolet absorbance before irradiation (ultraviolet absorbance after irradiation/ultraviolet absorbance before irradiation).

[SOLUTION TO PROBLEM]

The present invention, which can achieve the object described above, is as follows.

<Aspect 1>

A cosmetic composition, comprising a polar oil and a compound I having a structure of formula (I) below:

[Chem 2]

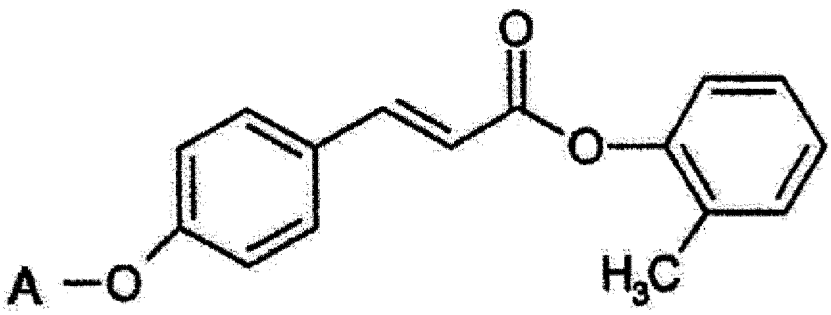

(I)

where -OA represents an alkoxy group,
wherein the compound I is 0.01 parts by mass or more and 50 parts by mass or less relative to a total of 100 parts by mass of the polar oil and the compound I.

<Aspect 2>

The composition according to Aspect 1, wherein the compound I is at least partially in a dissolved state.

<Aspect 3>

The composition according to Aspect 1 or 2, wherein the polar oil is at least one selected from the group consisting of an ester oil, an ether oil, a higher alcohol, and a fatty acid.

<Aspect 4>

The composition according to any one of Aspects 1 to 3, wherein the polar oil is at least one selected from the group consisting of bisethoxydiglycol cyclohexane-1,4-dicarboxylate, phenoxyethyl caprylate, PPG-30-BUTETH-30, diisopropyl sebacate, neopentyl glycol diheptanoate, diethylhexyl succinate, and isononyl isononanoate.

<Aspect 5>

The composition according to Aspect 4, wherein the polar oil is at least one selected from the group consisting of bisethoxydiglycol cyclohexane-1,4-dicarboxylate, phenoxyethyl caprylate, and PPG-30-BUTETH-30.

<Aspect 6>

The composition according to any one of Aspects 1 to 5, wherein a content of the polar oil is 0.1% by mass or more and 99.99% by mass or less.

<Aspect 7>

The composition according to any one of Aspects 1 to 6, which is a sunscreen cosmetic composition.

[ADVANTAGEOUS EFFECTS OF INVENTION]

According to the present invention, a cosmetic composition in which the effect of improving ultraviolet absorption ability in at least the UVA region is increased by ultraviolet irradiation or sunlight irradiation can be provided.

DESCRIPTION OF EMBODIMENTS

The embodiments of the present invention will be described below. Note that the present invention is not limited to the following embodiments, and various changes can be made within the scope of the spirit of the invention.

<<Cosmetic Composition>>

The cosmetic composition of the present invention (hereinafter also referred to simply as "the composition of the present invention") is:

a cosmetic composition, comprising a polar oil and a compound I having a structure of formula (I) below:

[Chem 3]

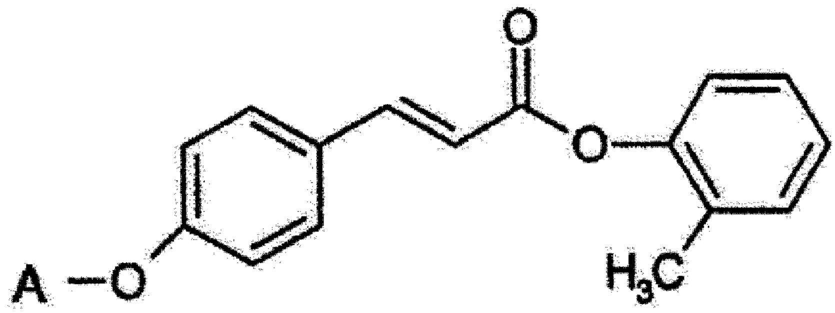

(I)

where -OA represents an alkoxy group,
wherein the compound I is 0.01 parts by mass or more and 50 parts by mass or less relative to a total of 100 parts by mass of the polar oil and the compound I.

The present inventors have discovered that by blending a polar oil and compound I into a composition at a specific composition ratio, the effect of improving ultraviolet absorption ability in at least the UVA region by ultraviolet irradiation or sunlight irradiation can be further increased.

The specific composition ratio of the polar oil and compound I is more specifically as follows: In the composition of the present invention, relative to a total of 100 parts by mass of the polar oil and compound I, the compound I may be 0.01 parts by mass or more, 0.05 parts by mass or more, 0.1 parts by mass or more, 0.5 parts by mass or more, 1.0 part by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, 9.5 parts by mass or more, 10 parts by mass or more, 15 parts by mass or more, or 20 parts by mass or more, and may be 50 parts by mass or less, 45 parts by mass or less, 40 parts by mass or less, 35 parts by mass or less, 30 parts by mass or less, 25 parts by mass or less, 20 parts by mass or less, 15 parts by mass or less, or 10 parts by mass or less.

<Polar Oil>

The composition of the present invention contains a polar oil. "Polar oil" as used herein refers to oils with high

polarity among oils usable in cosmetics, other than silicone oils, and refers to those having an IOB value of, for example, 0.10 or more, 0.11 or more, 0.12 or more, or 0.13 or more. Furthermore, the IOB value of the polar oil according to the present invention may be 2.0 or less, 1.5 or less, 1.0 or less, 0.50 or less, 0.45 or less, or 0.40 or less. The IOB value is an abbreviation of Inorganic/Organic Balance, is a value representing the ratio of the inorganic value to the organic value, and is an index indicating the degree of polarity of the organic compound. Specifically, the IOB value is expressed as IOB value = inorganic value / organic value. Regarding the "inorganic value" and the "organic value", depending on the type of atom or functional group, the "inorganic value" or "organic value" is set in a molecule, for example, one carbon atom has an "organic value" of 20 and one hydroxyl group has an "inorganic value" of 100, and by integrating the "inorganic value" or "organic value" of each atom and functional group in an organic compound, the IOB value of the organic compound can be calculated (for example, refer to Yoshio KODA, "Organic Conceptual Diagram - Basics and Applications," pp. 11-17, published by Sankyo Publishing, 1984).

In the present invention, the polar oil may be at least one selected from the group consisting of an ester oil, ether oil, higher alcohol, and fatty acid, each having an IOB value of 0.10 or more. Among these, a polar oil which is capable of dissolving the compound I described above is particularly preferable.

More specifically, the polar oil may be, for example, at least one selected from the group consisting of polyglyceryl-10 laurate (IOB value = 1.60), PEG-20 sorbitan cocoate (IOB value = 1.49), bisethoxydiglycol cyclohexane-1,4-dicarboxylate (IOB value = 1.03), phenoxyethyl caprylate (IOB value = 0.29), PPG-30-BUTETH-30 (IOB value = 0.94), diisopropyl sebacate (IOB value = 0.4), neopentyl glycol diheptanoate (IOB value = 0.33), diethylhexyl succinate (IOB value = 0.32), isononyl isononanoate (IOB value = 0.2), isopropyl myristate (IOB value = 0.18), isoamyl laurate (IOB value = 0.18), PPG-2 myristyl propionate (IOB value = 0.26), polyglyceryl-2 triisostearate (IOB value = 0.26), polyglyceryl-2 diisostearate (IOB value = 0.41), diethoxyethyl succinate (IOB value = 1.13), dioctyl adipate (IOB value = 0.29), diisopropyl adipate (IOB value = 0.55), dioctyl sebacate (IOB value = 0.24), diPPG-3 myristyl adipate (IOB value = 0.55), PPG-14 butyl (IOB value = 0.41), PPG-15 stearyl (IOB value = 0.32), PPG-3 myristyl (IOB value = 0.35), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), tri(caprylic acid/capric acid) glyceryl (IOB value = 0.33), alkyl benzoate (12 to 15 carbon atoms) (IOB value = 0.18), phenethyl benzoate (IOB value = 0.3), butyloctyl salicylate (IOB value = 0.43), and neopentyl glycol diheptanoate (IOB value = 0.33), but it is not limited thereto.

Furthermore, examples of ultraviolet absorbers that can be considered as the polar oil include ultraviolet absorbers having an IOB of 0.10 or more. Specific examples thereof include, but are not limited to, organic ultraviolet absorbers such as ethylhexyl methoxycinnamate, octocrylene, polysilicone-15, t-butylmethoxydibenzoylmethane, ethylhexyltriazone, bisethylhexyloxyphenolmethoxyphenyltriazine, diethylaminohydroxybenzoylhexyl benzoate, oxybenzone-3, methylenebisbenzotriazolyltetramethylbutylphenol, homosalate, and ethylhexyl salicylate. These ultraviolet absorbers can be used alone or in combination of two or more thereof.

Among the polar oils described above, from the viewpoint of facilitating the dissolution of the compound I according to the present invention, the polar oil is preferably at least one selected from the group consisting of bisethoxydiglycol cyclohexane-1,4-dicarboxylate (IOB 1.03), phenoxyethyl caprylate (IOB 0.29), PPG-30-BUTETH-30 (IOB 0.94), diisopropyl sebacate (IOB 0.4), neopentyl glycol diheptanoate (IOB 0.33), diethylhexyl succinate (IOB 0.32), and isononyl isononanoate (IOB 0.2), and from the viewpoint of further exerting the effects of the present invention, at least one selected from the group consisting of, for example, neopentyl glycol diheptanoate and diethylhexyl succinate is preferable. Further, from the viewpoint of further increasing the effect of improving ultraviolet absorption ability by ultraviolet irradiation or sunlight irradiation in both the UVA and UVB regions, the polar oil is preferably at least one selected from the group consisting of bisethoxydiglycol cyclohexane-1,4-dicarboxylate, phenoxyethyl caprylate, and PPG-30-BUTETH-30.

In the composition of the present invention, the content of polar oil is not particularly limited, and may be, relative to the entire cosmetic composition, for example, 0.1% by mass or more, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, 6.0% by mass or more, 6.5% by mass or more, 7.0% by mass or more, 7.5% by mass or more, 8.0% by mass or more, 8.5% by mass or more, 9.0% by mass or more, 9.5% by mass or more, 10% by mass or more, 15% by mass or more, 20% by mass or more, 25% by mass or more, 30% by mass or more, 35% by mass or more, 40% by mass or more, 45% by mass or more, 50% by mass or more, 55% by mass or more, 60% by mass or more, 65% by mass or more, 70% by mass or more,

75% by mass or more, 80% by mass or more, 85% by mass or more, or 90% by mass or more, and may be 99.99% by mass or less, 99% by mass or less, 98% by mass or less, 97% by mass or less, 96% by mass or less, or 95% by mass or less.

<Compound I>

Compound I according to the present invention has a structure of the following formula:

[Chem 4]

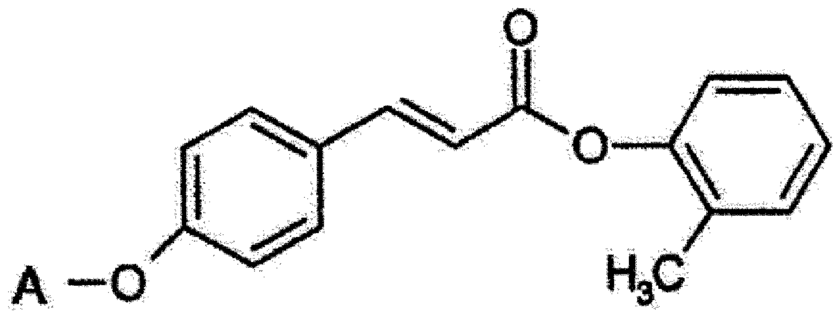

(I)

where -OA represents an alkoxy group.

In formula (I), -OA represents an alkoxy group, and more specific examples thereof include, but are not limited to, a methoxy group and an ethoxy group.

In the present invention, the compound I may have the same structure as the compound represented by formula I disclosed in Patent Literature 1.

Furthermore, in the composition of the present invention, from the viewpoint of further exerting the effects of the present invention, it is preferable that the compound I be at least partially in a dissolved state, in particular 50% by mass or more thereof be in a dissolved state, more particularly 90% by mass or more thereof be in a dissolved state, further particularly 99% by mass or more thereof be in a dissolved state, and most particularly 100% by mass thereof be in a dissolved state.

In the composition of the present invention, the content of compound I is not particularly limited, and may be, relative to the entire cosmetic composition, for example, 0.01% by mass or more, 0.05% by mass or more, 0.1% by mass or more, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, 6.0% by mass or more, 6.5% by mass or more, 7.0% by mass or more, 7.5% by mass or more, 8.0% by mass or more, 8.5% by mass or more, 9.0% by mass or more, 9.5% by mass or more, or 10% by mass or more, and may be 50% by mass or less, 40% by mass or less, 30% by mass or less, 20% by mass or less, 10% by mass or less, or 8.0% by mass or less.

<Applications and Forms of Composition of Present Invention>

The composition of the present invention can be suitably used as a cosmetic or a raw material thereof. Thus, the present invention in particular provides a sunscreen cosmetic composition.

The form of the composition of the present invention is not particularly limited, and may be, for example, an oil-in-water emulsified cosmetic composition, a water-in-oil emulsified cosmetic composition, or an oil-based oil cosmetic composition.

<Other Components>

The cosmetic composition of the present invention may further contain, in addition to the above-mentioned components, arbitrary aqueous components or other components which can be used in the cosmetics field. As the other components, for example, other components such as an oil other than the polar oil described above, ultraviolet scattering agent particles, ultraviolet absorbers, surfactants, moisturizers, sequestering agents, natural and synthetic polymers, water-soluble and oil-soluble polymers, various extracts, colorants such as organic dyes, preservatives, antioxidants,

pigments, thickeners, pH adjusters, fragrances, cooling agents, antiperspirants, disinfectants, skin activators, other chemicals, and various powders may be further contained, but the other components are not limited thereto.

The other components will be exemplified below, but the present invention is not limited thereto.

(Oil Other Than Polar Oil)

The composition of the present invention may further contain an oil other than the polar oil described above as long as the effects of the present invention are not impaired thereby. In this case, relative to a total of 100 parts by mass of the polar oil and the oil other than the polar oil, the oil other than polar oil may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less, and may be 0.1 parts by mass or more, 0.5 parts by mass or more, 1.0 part by mass or more, 5.0 parts by mass or more, or 10 parts by mass or more.

In the present invention, examples of the oil other than the polar oil include, but are not limited to, silicone oils and hydrocarbon oils.

(i) Silicone Oil

"Silicone oil" refers to an oil having a main skeleton composed of siloxane bonds among oils usable in cosmetics. Note that oils "having a main skeleton composed of siloxane bonds" and being usable as the "ultraviolet absorber" described above are excluded from the "silicone oil" referred to herein.

In the present invention, the silicone oil may be a volatile silicone oil or a nonvolatile silicone oil.

In the present invention, the boiling point at one atmosphere (101.325 kPa) can be used as a guideline for "volatile." From the viewpoint of suitably dispersing the ultraviolet scattering agent, the boiling point is preferably 250 °C or lower, 240 °C or lower, or 230 °C or lower, and is preferably 80 °C or higher, 100 °C or higher, 120 °C or higher, 150 °C or higher, or 160 °C or higher. Furthermore, in the present disclosure, "nonvolatile" is intended to mean that the volatile content is 5% or less when a sample is placed in a sufficiently large flat-bottomed dish and allowed to stand at 105 °C for 3 hours.

Further, the silicone oil may be an acyclic silicone oil (i.e., a linear silicone oil) or a cyclic silicone oil.

Specific examples of the silicone oil include, but are not limited to, linear silicone oils such as polydimethylsiloxane (dimethicone), methylphenylpolysiloxane, and methylhydrogenpolysiloxane, cyclic silicone oils such as octamethylcyclotetrasiloxane, decamethylcyclopentasiloxane, and dodecamethylcyclohexasiloxane, and mixtures of two or more thereof.

(ii) Hydrocarbon Oil

In the present invention, "hydrocarbon oil" refers to a hydrocarbon oil other than the polar oil described above.

The hydrocarbon oil may be a volatile hydrocarbon oil or a nonvolatile hydrocarbon oil.

Specific examples of the hydrocarbon oil include, but are not limited to, decane, dodecane, isododecane, isohexadecane, liquid paraffin, squalane, squalene, paraffin, and mixtures of two or more thereof.

<Ultraviolet Scattering Agent Particles>

From the viewpoint of, for example, improving the ultraviolet protection effect, the composition of the present invention may further contain ultraviolet scattering agent particles.

The ultraviolet scattering agent particles are not particularly limited, and may be, for example, at least one selected from the group consisting of titanium oxide, zinc oxide, iron oxide, and cerium oxide.

The ultraviolet scattering agent particles may be subjected to a hydrophobization treatment. The hydrophobization treatment may be performed using a known surface treatment agent for hydrophobization, and examples thereof include a fluorine compound treatment, silicone treatment, silicone resin treatment, pendant treatment, silane coupling agent treatment, titanium coupling agent treatment, oil treatment, N-acylated lysine treatment, polyacrylic acid treatment, metal soap treatment, amino acid treatment, inorganic compound treatment, plasma treatment, mechanochemical treatment, silane compound treatment, and silazane compound treatment. Among these treatments, from the viewpoint of dispersion stability, a treatment with silicone or silicone resin, treatment with silane compound or silazane compound, and treatment with metal soap such as aluminum isostearate are preferable.

The average primary particle diameter of the ultraviolet scattering agent particles is not particularly limited, and may be, for example, 5 nm or more, 10 nm or more, or 15 nm or more, and may be 200 nm or less, 100 nm or less, or 50 nm or less. Note that the "average primary particle diameter" may be determined as the circle equivalent diameter of the projected area of the primary particles in an SEM image.

The shape of the ultraviolet scattering agent particles is not particularly limited, and examples thereof include spherical, plate-like, rod-like, spindle-like, needle-like, and irregular shapes.

<Ultraviolet Absorber>

From the viewpoint of, for example, improving the ultraviolet protection effect, the composition of the present invention may further contain an ultraviolet absorber.

The ultraviolet absorber is not particularly limited, and examples thereof include:

ultraviolet absorbers having a camphor group such as terephthalylidene dicanfursulfonic acid;
ultraviolet absorbers having a sulfonic acid group such as phenylbenzimidazole sulfonic acid;
ultraviolet absorbers having a triazine group such as bisethylhexyloxyphenolmethoxyphenyltriazine and ethylhexyltriazone;
ultraviolet absorbers containing a cinnamic acid such as cresyl methoxycinnamate;
ultraviolet absorbers having a silicone group such as methylbis(trimethylsiloxy)silylisopentyl trimethoxycinnamate and drometrizole trisiloxane;
ultraviolet absorbers having a carbonyl group such as t-butylmethoxydibenzoylmethane, benzophenone-3, and benzophenone-5;
ultraviolet absorbers having a benzotriazole group such as methylenebisbenzotriazolyltetramethylbutylphenol, and
ultraviolet absorbers having an amino group such as diethylaminohydroxybenzoylhexyl benzoate.

Note that these listed ultraviolet absorbers, if corresponding to the "polar oil" described above, are regarded as the "polar oil".

EXAMPLES

The present invention will be described in more detail below with reference to the Examples, but the present invention is not limited thereto.

<<Comparative Example 1 and Examples 1 to 12»

The compositions of Comparative Example 1 and Examples 1 to 6 were prepared based on the compositions in Table 1 below. Furthermore, the compositions of Examples 7 to 12 were prepared based on the compositions shown in Table 2 below.

Note that the compound represented by the following structural formula was used as compound I:

[Chem 5]

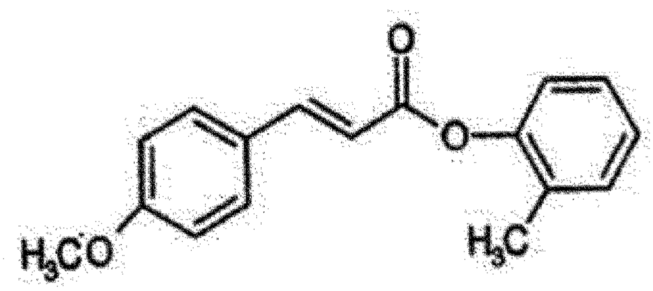

«Evaluation of Effect of Improving Ultraviolet Absorption Ability by Ultraviolet Irradiation or Sunlight Irradiation»

Samples of the prepared compositions of Examples and Comparative Examples were each applied to a PMMA plate, and irradiated with simulated sunlight using a device for applying simulated sunlight (SUNTEST XLS+, device manufactured by ATLAS) (hereinafter referred to as "sun-testing"). Specifically, sun-testing was conducted under the following conditions:

Irradiation time: 2 hours
Surface temperature: 50 °C

Irradiation output: 3860 $KJ/m^2$

The ultraviolet absorption wavelength of the sample before sun-testing and the ultraviolet absorption wavelength of the sample after sun-testing were measured.

The samples of Examples and Comparative Examples each showed a change in the ultraviolet absorption wavelengths before and after sun-testing. The absorbances before and after irradiation at 360 nm (UVA region) were then determined for each sample as the improvement rate of ultraviolet absorption ability by sunlight irradiation (hereinafter also simply referred to as "improvement rate") according to the following formula:

$$\text{Improvement rate} = (\text{absorbance after irradiation} / \text{absorbance before irradiation}) \times 100\%$$

Furthermore, the improvement rate of Comparative Example 1 was defined as "1 (reference)", and the ratio of the improvement rate of each Example was determined as a "relative improvement rate" (improvement rate of each Example / improvement rate of Comparative Example 1). The results are shown in Tables 1 and 2.

Regarding each of the samples of Examples and Comparative Examples, the presence or absence of an increase in absorption in the UVA region (wavelength 320 to 400 nm) of ultraviolet absorption wavelengths after sun-testing was examined. Similarly, the presence or absence of an increase in absorption in the UVB region (wavelength 290 to 320 nm) of ultraviolet absorption wavelengths after sun-testing was examined. The results are shown in Tables 1 and 2.

[Table 1]

| (Table 1) | | | Comparative Example 1 | Example 1 | Example 2 | Example 3 | Example 4 | Example 5 | Example 6 |
|---|---|---|---|---|---|---|---|---|---|
| Composition | Compound I (% by mass) | | 20.0 | 5.0 | 5.0 | 5.0 | 5.0 | 5.0 | 5.0 |
| | Polar oil (% by mass) | Isononyl isononanoate | 5.0 | 19.0 | 47.5 | 95.0 | 15.8 | 47.5 | 76 |
| | | Bisethoxydiglycol cyclohexane-1,4-dicarboxylate | - | - | - | - | - | - | - |
| | | Phenoxyethyl caprylate | - | - | - | - | - | - | - |
| | | PPG-30-BUTETH-30 | - | - | - | - | - | - | - |
| | | Diisopropyl sebacate | - | - | - | - | - | - | - |
| | | Neopentyl glycol diheptanoate | - | - | - | - | - | - | - |
| | | Diethylhexyl succinate | - | - | - | - | - | - | - |
| | Oil other th an polar oil (% by mass) | Silicone D5 (volatile) | 23.5 | 76.0 | 47.5 | - | - | - | - |
| | | Silicone 6T (nonvolatile) | - | - | - | - | 79.2 | 47.5 | 19.0 |
| Parts by mass of compound I relative to total 100 parts by mass of polar oil and compound I | | | 80.0 | 20.8 | 9.5 | 5.0 | 24.0 | 9.5 | 6.2 |
| Evaluation o f UV absor ption ability improveme nt effect by sunlight irradiation | Relative improvement rate | | 1 | 1.25 | 1.41 | 5.79 | 2.05 | 2.26 | 1.83 |
| | Presence/absence of increased absorption in UVA region | | Present | Present | Present | Present | Present | Present | Present |
| | Presence/absence of increased absorption in UVB region | | Absent | Absent | Absent | Absent | Absent | Absent | Absent |

[Table 2]

| (Table 2) | | | Example 7 | Example 8 | Example 9 | Example 10 | Example 11 | Example 12 |
|---|---|---|---|---|---|---|---|---|
| Composition | Compound I (% by mass) | | 5.0 | 5.0 | 5.0 | 5.0 | 5.0 | 5.0 |
| | Polar oil (% by mass) | Isononyl isononanoate | - | - | - | - | - | - |
| | | Bisethoxydiglycol cyclohexane-1,4-dicarboxylate | 95.0 | - | - | - | - | - |
| | | Phenoxyethyl caprylate | - | 95.0 | - | - | - | - |
| | | PPG-30-BUTETH-30 | - | - | 95.0 | - | - | - |
| | | Diisopropyl sebacate | - | - | - | 95.0 | - | - |
| | | Neopentyl glycol diheptanoate | - | - | - | - | 95.0 | - |
| | | Diethylhexyl succinate | - | - | - | - | - | 95.0 |
| Parts by mass of compound I relative to total 100 parts by mass of polar oil and compound I | | | 5.0 | 5.0 | 5.0 | 5.0 | 5.0 | 5.0 |
| Evaluation of UV absorption ability improve ment effect by sunlight irradiation | Relative improvement rate | | 3.53 | 4.73 | 2.43 | 2.49 | 80.4 | 61.8 |
| | Presence/absence of increased absorption in UVA region | | Present | Present | Present | Present | Present | Present |
| | Presence/absence of increased absorption in UVB region | | Present | Present | Present | Absent | Absent | Absent |

As is clear from the results of Tables 1 and 2, it was discovered that, as compared to Comparative Example 1, all of the compositions of Examples 1 to 12 had an increased effect of improving ultraviolet absorption ability in at least the UVA region.

## Claims

1. A cosmetic composition, comprising a polar oil and a compound I having a structure of formula (I) below:

[Chem 1]

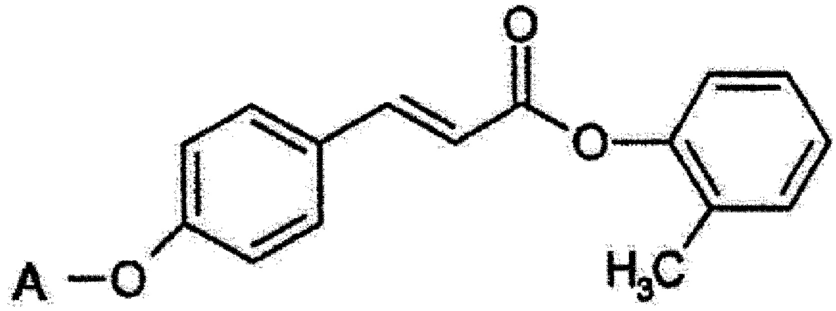

(I)

where -OA represents an alkoxy group,
wherein the compound I is 0.01 parts by mass or more and 50 parts by mass or less relative to a total of 100 parts by mass of the polar oil and the compound I.

2. The composition according to claim 1, wherein the compound I is at least partially in a dissolved state.

3. The composition according to claim 1 or 2, wherein the polar oil is at least one selected from the group consisting of an ester oil, an ether oil, a higher alcohol, and a fatty acid.

4. The composition according to any one of claims 1 to 3, wherein the polar oil is at least one selected from the group consisting of bisethoxydiglycol cyclohexane-1,4-dicarboxylate, phenoxyethyl caprylate, PPG-30-BUTETH-30, diisopropyl sebacate, neopentyl glycol diheptanoate, diethylhexyl succinate, and isononyl isononanoate.

5. The composition according to claim 4, wherein the polar oil is at least one selected from the group consisting of bisethoxydiglycol cyclohexane-1,4-dicarboxylate, phenoxyethyl caprylate, and PPG-30-BUTETH-30.

6. The composition according to any one of claims 1 to 5, wherein a content of the polar oil is 0.1% by mass or more and 99.99% by mass or less.

7. The composition according to any one of claims 1 to 6, which is a sunscreen cosmetic composition.

## INTERNATIONAL SEARCH REPORT

International application No.
**PCT/JP2022/036513**

**A. CLASSIFICATION OF SUBJECT MATTER**

***A61K 8/37***(2006.01)i; ***A61K 8/86***(2006.01)i; ***A61Q 17/04***(2006.01)i
FI: A61K8/37; A61K8/86; A61Q17/04

According to International Patent Classification (IPC) or to both national classification and IPC

**B. FIELDS SEARCHED**

Minimum documentation searched (classification system followed by classification symbols)

A61K8/37; A61K8/86; A61Q17/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN); Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | WO 2009/041098 A1 (LEAD CHEMICAL CO., LTD.) 02 April 2009 (2009-04-02) claims, paragraphs [0005], [0008], [0016], [0017] | 1-3, 6-7 |
| Y | | 1-7 |
| Y | JP 2018-083785 A (KRACIE HOME PRODUCTS, LTD.) 31 May 2018 (2018-05-31) claims, paragraphs [0001], [0028], [0049] | 1-7 |
| Y | JP 2021-095337 A (MILBON CO., LTD.) 24 June 2021 (2021-06-24) claims, paragraphs [0007], [0026], [0048] | 1-4, 6-7 |
| A | JP 2020-193190 A (IKEDA BUSSAN CO., LTD.) 03 December 2020 (2020-12-03) paragraph [0025] | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/036513**

| Patent document cited in search report | Publication date (day/month/year) | Patent family member(s) | Publication date (day/month/year) |
|---|---|---|---|
| WO 2009/041098 A1 | 02 April 2009 | US 2010/0239508 A1<br>claims, paragraphs [0029], [0042], [0075]-[0085]<br>EP 2192106 A1<br>AU 2008305887 A1<br>CA 2701660 A1<br>CN 101808976 A<br>KR 10-2010-0069662 A | |
| JP 2018-083785 A | 31 May 2018 | (Family: none) | |
| JP 2021-095337 A | 24 June 2021 | (Family: none) | |
| JP 2020-193190 A | 03 December 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009041098 A


### Non-patent literature cited in the description


- **YOSHIO KODA.** Organic Conceptual Diagram - Basics and Applications. Sankyo Publishing, 1984, 11-17